# EUROPÄISCHE PATENTSCHRIFT

(11) **EP 0 970 077 B1**
(45) Veröffentlichungstag und Bekanntmachung des Hinweises auf die Patenterteilung: **14.05.2003**
(21) Anmeldenummer: 98916974.3
(22) Anmeldetag: 17.03.1998
(51) Int. Cl.: C07D 417/14, A61K 31/425, C07D 417/12

(54) **VERBESSERTES VERFAHREN ZUR HERSTELLUNG VON THIAZOLIDINDIONEN UND NEUE THIAZOLIDINDIONE**
IMPROVED METHOD FOR PRODUCING THIAZOLIDINEDIONES, AND NEW THIAZOLIDINEDIONES
PROCEDE AMELIORE DE PRODUCTION DE THIAZOLIDINEDIONES ET NOUVELLES THIAZOLIDINEDIONES

(30) Priorität: 20.03.1997 DE 19711616
(43) Veröffentlichungstag der Anmeldung: 12.01.2000
(73) Patentinhaber: Roche Diagnostics GmbH, 68298 Mannheim (DE)
(72) Erfinder: WOLFF, Hans-Peter, D-69469 Weinheim (DE); WITTE, Ernst-Christian, D-68165 Mannheim (DE); KÜHNLE, Hans-Frieder, D-69469 Weinheim (DE)
(74) Vertreter: Witte, Hubert, Dr.
(86) Internationale Anmeldenummer: EP9801535
(87) Internationale Veröffentlichungsnummer: WO98042704

(56) Entgegenhaltungen:
- EP-A- 0 306 228
- EP-A- 0 332 331
- EP-A- 0 454 501
- WO-A-93/13095
- WO-A-94/27995

## Beschreibung

In der Anmeldung WO 94/27995 sind Thiazolidindion-Derivate der allgemeinen Formel I beschrieben, 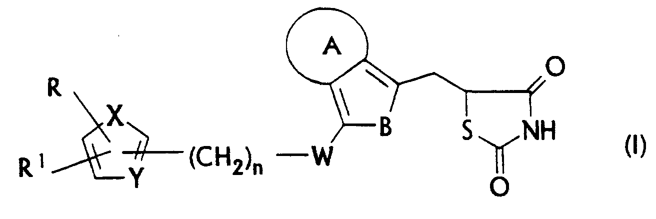 worin
- A: einen carbocyclischen Ring mit 5 oder 6 Kohlenstoffatomen oder einen heterocyclischen Ring mit max. 4 Heteroatomen darstellt. wobei die Heteroatome gleich oder verschieden sein können und Sauerstoff. Stickstoff oder Schwefel bedeuten, und die Heterocyclen gegebenenfalls an einem oder mehreren Stickstoffatomen ein Sauerstoffatom tragen können,
- B: -CH=CH-, -N=CH-, -CH=N-, O oder S,
- W: CH₂, O, CH(OH), CO oder -CH=CH-,
- X: S, O oder NR², wobei der Rest R² Wasserstoff oder C₁-C₆ Alkyl ist.
- Y: CH oder N
- R: Naphthyl, Pyridyl, Furyl, Thienyl oder Phenyl, das gegebenenfalls mit C₁-C₃ Alkyl, CF₃, C₁-C₃ Alkoxy, F, Cl, oder Brom mono- oder disubstituiert ist,
- R¹: Wasserstoff oder C₁-C₆ Alkyl und
- n: 1-3
bedeutet, sowie deren Tautomere, Enantiomere , Diastereomere und physiologisch verträglichen Salze.

Verbindungen der allgemeinen Formel I können mit Basen Salze bilden, da sie am Thiazolidindion-Ring eine saure NH-Gruppe besitzen. Geeignete pharmazeutische Salze sind beispielsweise Alkalisalze wie die Lithium-, Natrium- oder Kaliumsalze, Erdalkalisalze wie die Calcium- oder Magnesiumsalze, andere Metallsalze wie z.B. Aluminiumsalze, Ammoniumsalze oder Salze mit organischen Basen wie z.B. Diethanolamin, Ethylendiamin, Diisopropylamin und andere. Besonders bevorzugt ist das Natriumsalz. Die Herstellung der Salze erfolgt beispielsweise durch Behandeln der Verbindungen der allgemeinen Formel (I) mit einer stöchiometrischen Menge der entsprechenden Base in an sich bekannter Weise.

Die Herstellung der Verbindungen erfolgt üblicherweise durch nachträglichen Aufbau des Thiazolidindion-Ringsystems nach einem an sich bekannten Verfahren über alpha-halogenierte Carbonsäuren. Zur Herstellung dieser Carbonsäuren wird eine aromatische Aminogruppe diazotiert und das Diazoniumsalz mit Acrylsäureethylester in Gegenwart von Halogenwasserstoffsäure und Kupfersalzen umgesetzt. Dieses Verfahren besitzt für die Herstellung von Mengen im Multi-kg-Maßstab einige Nachteile in Bezug auf Sicherheit, Upscaling, Handling und Syntheseaufwand. Unter anderem muß bei der Umsetzung der aromatischen Amine zu den alpha-halogenierten Carbonsäuren diazotiert und mit giftigem Acrylsäureester umgesetzt werden, wobei diese Reaktion mit unbefriedigenden Ausbeuten verläuft. Ausserdem ist die Übertragung dieser Reaktion in einen grösseren Maßstab aus Sicherheits- und Umweltschutzgründen äußerst problematisch.

Ein weiteres erfindungsgemäßes Herstellungsverfahren besteht in der katalytischen Hydrierung von Verbindungen der allgemeinen Formel II, 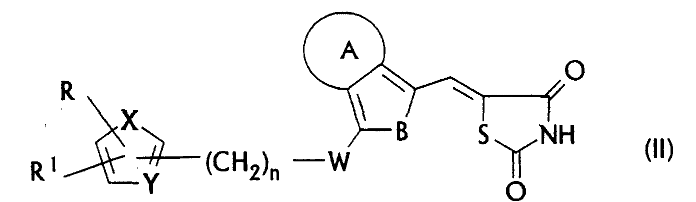 worin A, B, W, X, Y, R, R¹ und n die oben angegebenen Bedeutungen besitzen.

Die katalytische Hydrierung gestaltet sich jedoch sehr aufwendig. Insbesondere bei den Vebindungstypen, die zusätzlich zu dem im Thiazolidindion-Ring enthaltenen Schwefel ein weiteres Schwefelatom im Molekül tragen, ist mit einer Vergiftung des Katalysators durch Schwefel zu rechnen, der zu sehr langen Reaktionszeiten führt und eine mehrfache Erneuerung des Katalysators notwendig macht.

Aus der Literatur ist ein weiteres Verfahren zur Reduktion von Verbindungen der allgemeinen Formel II bekannt, das darin besteht, daß man als Reduktionmittel Magnesium verwendet [z.B. C.C.Cantello et al. in J. Med. Chem. 37, 3977 (1994)].

Durch diese Methode können die beim katalytischen Verfahren auftretenden Störungen durch den im Molekül enthaltenen Schwefel umgangen werden, jedoch tritt im Falle der oben gennannten Verbindungen der allgemeinen Formel II eine partielle Reduktion des fünfgliedrigen ungesättigten Heterocyclus ein, die zu schwer abtrennbaren Verunreinigungen der gewünschten Produkte führt.

Überraschend wurde nun gefunden, daß sich Verbindungen der allgemeinen Formel II nach der zuletzt geschilderten Methode glatt und in hoher Reinheit reduzieren lassen, ohne daß eine partielle Reduktion der Doppelbindungen des fünfgliedrigen heterocyclischen Ringsystems stattfindet, wenn man anstelle von Magnesium als reduzierendem Agenz metallisches Aluminium verwendet, wobei das Aluminium vorteilhafterweise durch Behandeln der Oberfläche mit Salzen edlerer Metalle aktiviert wird.

Das Verfahren findet insbesondere Anwendung auf eine Auswahl besonders wertvoller Unterklassen der allgemeinen Formel I, die nachfolgend unter der allgemeinen Formel III zusammengefasst sind.

Gegenstand der vorliegenden Erfindung ist daher ein neues Verfahren zur Herstellung von Verbindungen der allgemeinen Formel (III), 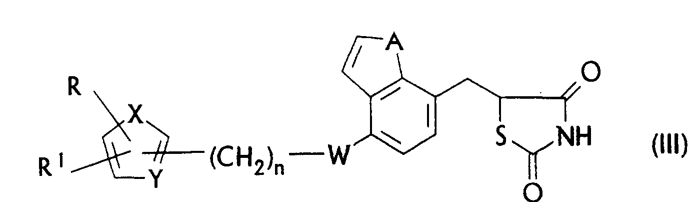 worin
- A: CH=CH oder S
- W: O
- X: S, O oder NR², wobei der Rest R² Wasserstoff oder C₁-C₆ Alkyl ist,
- Y: CH oder N,
- R: Naphthyl, Thienyl oder Phenyl, das gegebenenfalls mit C₁-C₃ Alkyl, CF₃, C₁-C₃ Alkoxy, F, Cl oder Brom mono- oder disubstituiert ist,
- R¹: Wasserstoff oder C₁-C₆ Alkyl und
- n: 1-3 bedeutet,
indem man eine Verbindung der allgemeinen Formel IV, 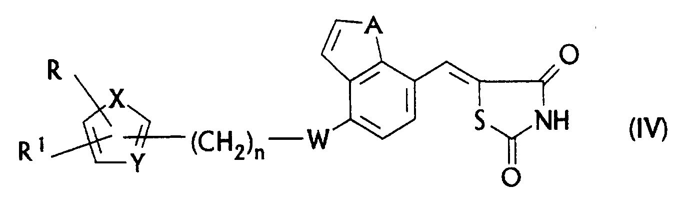 in welcher A, W, X, Y, R, R₁ und n die oben angegebene Bedeutung besitzen, mit aktiviertem Aluminium in einem protischen Lösungsmittel reduziert. Die Aktivierung der Oberfläche des Aluminiums kann durch Behandeln mit Metallsalzen erfolgen, die in der Spannungsreihe über Aluminium stehen. Besonders geeignet ist eine verdünnte Lösung von Quecksilberchlorid. Das Aluminium kann in Form von Spänen, Grieß oder Pulver eingesetzt werden. Als protische Lösungsmittel kommen bevorzugt niedere Alkohole, insbesondere Methanol, aber auch Wasser infrage. Zur Verbesserung der Löslichkeit können aprotische organische, mit Alkoholen oder Wasser mischbare Lösungsmittel zugesetzt oder zum überwiegenden Anteil verwendet werden. Die Reaktion wird bei 0 - 80°C, bevorzugt bei Raumtemperatur oder leicht erhöhter Temperatur bis 40°C durchgeführt.

Die Erfindung betrifft auch eine neue Verbindung der allgemeinen Formel I, die nicht in der Anmeldung WO 94/27995 beschrieben ist und die im Vergleich zu den in dieser Anmeldung beschriebenen Verbindungen ein überraschend besseres Wirkprofil bei der Behandlung des Diabetes mellitus zeigt. Dies ist die folgende Verbindung:
5-{4-[2-(5-Methyl-2-(thien-2-yl)-oxazol-4-yl)-ethoxy]-benzo[*b*]thiophen-7-ylmethyl}-thiazolidin-2.4-dion
deren Tautomere, Enantiomere, Diastereomere und physiologisch verträgliche Salze.

Ferner sind Gegenstand der Erfindung Arzneimittel, die vorstehend aufgeführte Verbindung als Wirkstoff enthalten, zur Behandlung der Diabetes mellitus. Die Herstellung der Arzneimittel und deren Verwendung erfolgt nach üblichen, in WO 94/27995 beschriebenen Methoden.

Die nachfolgenden Beispiele sollen die neue Methode zur Herstellung der Verbindung der Formel (III) erläutern, ohne die Methode auf die genannte speziellen Fälle zu beschränken. Die Herstellung der Verbindungen der allgemeinen Formel IV erfolgt nach den in WO 94/27995 angegebenen Verfahren.

### Beispiel 1

### 5-{4-[2-(5-Methyl-2-phenyloxazol-4-yl)-ethoxy]-benzor[b]thiophen-7-ylmethyl}thiazolidin-2.4-dion

### a) Herstellung von aktiviertem Aluminium

5 ml einer gesättigten Lösung von Quecksilber-II-chlorid in Ethanol werden mit Ethanol auf 50 ml verdünnt und mit 10 g Aluminium-Nadeln kurz geschüttelt. Dann wird die Lösung abdekantiert und die Nadeln 2 mal mit Ethanol, einmal mit Diethylether und einmal mit Tetrahydrofuran gewaschen.

### b) Titelverbindung

Eine Lösung von 2,03 g (4,3 mmol) 5-{4-[2-(5-Methyl-2-phenyloxazol-4-yl)-ethoxy]-benzo[*b*]thiophen-7-ylmethyliden}-thiazolidin-2.4-dion (Fp.204-207°C) in 130 ml Tetrahydrofuran wird mit 10 g aktivierten Aluminium-Nadeln und 1 ml Wasser versetzt. Dann erhitzt man 50 min. unter Rühren auf 50°C und filtriert die festen Anteile ab. Das Filtrat wird eingedampft und der Rückstand mit einem Gemisch von 88 Vol.-Teilen Toluol, 10 Vol.-Teilen 2-Butanon und 2 Vol.-Teilen Eisessig an Kieselgel chromatographiert. Ausbeute: 1.65 g (83%), Fp.204-206°C.

### Beispiel 2

In analoger Weise werden erhalten:

### a) 5-{4-[2-(5-Methyl-2-(thien-2-yl)-oxazol-4-yl)-ethoxy]-benzo[b]thiophen-7-ylmethyl}-thiazolidin-2.4-dion

Ausbeute: 57 %; Fp: 115-117 °C
aus
5-{4-[2-(5-Methyl-2-(thien-2-yl)-oxazol-4-yl)-ethoxy]-benzo[*b*]thiophen-7-ylmethyliden}-thiazolidin-2.4-dion (Fp. 229-234°C).

### b) 5-{4-[2-(5-Methyl-2-phenyloxazol-4-yl)-ethoxy]-naphth-1-ylmethyl}thiazolidin-2.4-dion

Ausbeute: 76 %; Fp: 187-191 °C
aus
5-{4-[2-(5-Methyl-2-phenyl-oxazol-4-yl)-ethoxy]-naphth-1-ylmethyliden}thiazolidin-2.4-dion (Fp: 252-254 °C).

### c) 5-{4-[2-(5-Methyl-2-(4-fluorphenyloxazol)-4-yl)-ethoxy]-benzo[b]thiophen-7-ylmethyl}-thiazolidin-2.4-dion

Ausbeute: 81 %. Fp 205 °C
aus
5-{4-[2-(5-Methyl-2-(4-fluorphenyloxazol)-4-yl)-ethoxy]-benzo[*b*]thiophen-7-yl-methyliden}-thiazolidin-2.4-dion (Fp. 240 °C).

### d) 5-{4-[2-(5-Methyl-2-(4-chlorphenyloxazol)-4-yl)-ethoxy]-benzo[b]thiophen-7-ylmethyl}-thiazolidin-2.4-dion

Ausbeute 60 %. Fp. 208 °C
aus
5-{4-[2-(5-Methyl-2-(4-chlorphenyloxazol)-4-yl)-ethoxy]-benzo[*b*]thiophen-7-yl-methyliden}-thiazolidin-2.4-dion (Fp. 270 °C).

### e) 5-{4-[2-(5-Methyl-2-(4-trifluormethylphenyloxazol)-4-yl)-ethoxy]-benzo[b]thiophen-7-ylmethyl}-thiazolidin-2.4-dion

Ausbeute 57 %, Fp. 191 °C
aus
5-{4-[2-(5-Methyl-2-(4-trifluormethylphenyl)oxazol-4-yl)-ethoxy]-benzo[*b*]thiophen-7-yl-methyliden}-thiazolidin-2.4-dion (Fp. 260 °C).

### f) 5-{4-[2-(5-Methyl-2-(2.4-difluorphenyloxazol)-4-yl)-ethoxy]-benzo[b]thiophen-7-ylmethyl}-thiazolidin-2.4-dion

Ausbeute 78 %, Fp. 189 °C
aus
5-{4-[2-(5-Methyl-2-(2.4-difluorphenyloxazol)-4-yl)-ethoxy]-benzo[*b*]thiophen-7-yl-methyliden}-thiazolidin-2.4-dion
(amorphes Pulver, Fp. ab 255 °C).

## Patentansprüche

1. Verfahren zur Herstellung von Verbindungen der Formel III 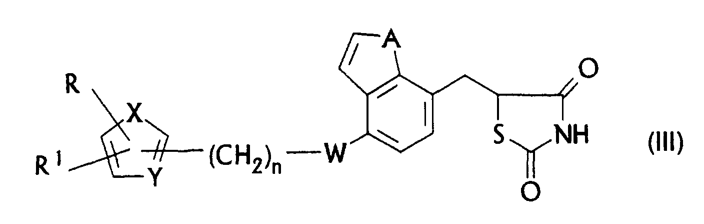 worin
A CH=CH oder S
W O
X S, O oder NR², wobei der Rest R² Wasserstoff oder C₁-C₆ Alkyl ist,
Y CH oder N,
R Naphthyl, Thienyl oder Phenyl, das gegebenenfalls mit C₁-C₃ Alkyl, CF₃, C₁-C₃ Alkoxy, F, Cl oder Brom mono- oder disubstituiert ist,
R¹ Wasserstoff oder C₁-C₆ Alkyl und
n 1-3 bedeutet,
**dadurch gekennzeichnet, daß** man eine Verbindung der Formel IV 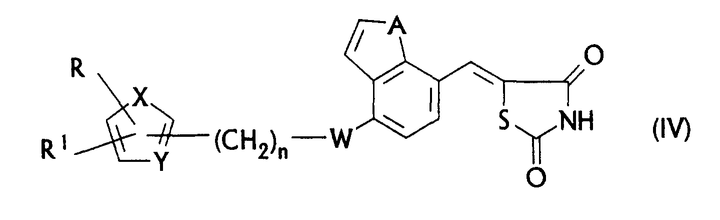 in welcher A, W, X, Y, R, R₁ und n die oben angegebene Bedeutung besitzen,
mit metallischem Aluminium in einem protischen Lösungsmittel reduziert.

2. Verfahren gemäß Anspruch 1, **dadurch gekennzeichnet, daß** das eingesetzte metallische Aluminium durch Behandeln der Oberflächen mit Salzen edler Metalle aktiviert wird.

3. Verfahren gemäß Anspruch 1 oder 2, **dadurch gekennzeichnet, daß** die Aktivierung des Aluminiums mit einer Quecksilberchlorid-Lösung erfolgt.

4. 5-{4-[2-(5-Methyl-2-(thien-2-yl)-oxazol-4-yl)-ethoxy]-benzo[*b*]thiophen-7-ylmethyl}-thiazolidin-2.4-dion
sowie deren Tautomere, Enantiomere, Diastereomere und physiologisch verträgliche Salze.

5. Arzneimittel enthaltend neben üblichen Träger- und Hilfsstoffen eine Verbindung gemäß Anspruch 4.

6. Verwendung von Verbindungen gemäß Anspruch 4 zur Herstellung von Arzneimitteln zur Behandlung des Diabetes mellitus.

## Claims

1. A process for the production of compounds of formula III 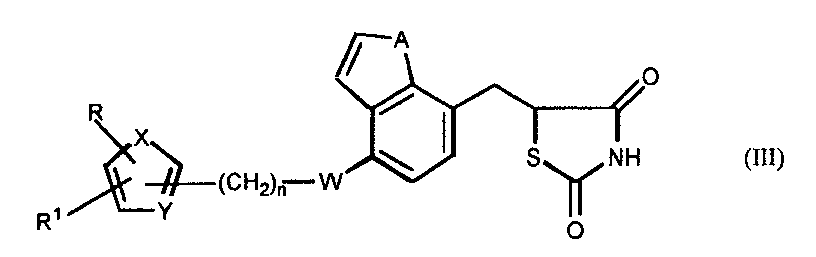 wherein
A signifies CH=CH or S
W signifies O
X signifies S, O or NR² in which the residue R² is hydrogen or C₁-C₆ alkyl,
Y signifies CH or N
R signifies naphthyl, thienyl or phenyl which is optionally mono- or disubstituted with C₁-C₃ alkyl, CF₃, C₁-C₃ alkoxy, F, Cl or bromine,
R¹ signifies hydrogen or C₁-C₆ alkyl and
n signifies 1-3,
**characterized by** reducing a compound of formula IV 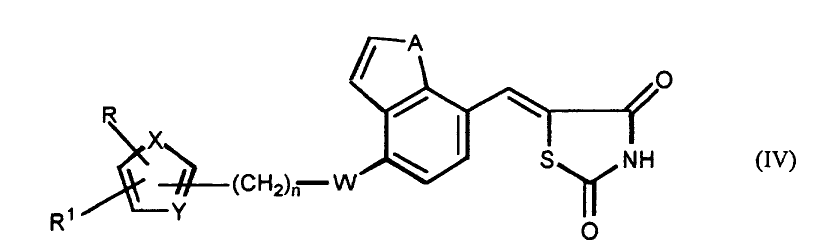 in which A, W, X, Y, R, R₁ and n have the significance given above, with metallic aluminium in a protic solvent.

2. A process in accordance with claim 1, **characterized in that** the metallic aluminium used is activated by treatment of the surface with salts of noble metals.

3. A process in accordance with claim 1 or 2, **characterized in that** the activation of the aluminium is effected with a mercuric chloride solution.

4. 5-{4-[2-(5-Methyl-2-(thien-2-yl)-oxazol-4-yl)-ethoxy]-benzo[b]thiophen-7-ylmethyl}-thiazolidine-2,4-dione as well as tautomers, enantiomers, diastereomers and physiologically compatible salts thereof.

5. A medicament containing a compound in accordance with claim 4 in addition to conventional carriers and adjuvants.

6. The use of compounds in accordance with claim 4 for the production of medicaments for the treatment of diabetes mellitus.

## Revendications

1. Procédé pour la préparation de composés de formule III 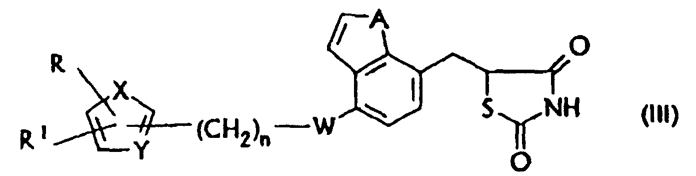 dans laquelle
A représente CH=CH ou S
W représente O
X représente S, O ou NR², le radical R² étant un atome d'hydrogène ou un groupe alkyle en C₁-C₆
Y représente CH ou N,
R représente un groupe naphtyle, thiényle ou phényle, qui est éventuellement mono- ou disubstitué par un ou des atomes de F, Cl ou Br ou groupes alkyle en C₁-C₃, CF₃, alcoxy en C₁-C₃,
R¹ représente un atome d'hydrogène ou un groupe alkyle en C₁-C₆ et
n représente 1-3,
**caractérisé en ce qu'**on réduit un composé de formule IV 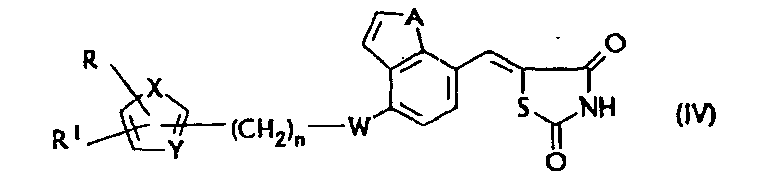 dans laquelle A, W, X, Y, R, R¹ et n ont les significations données ci-dessus, avec de l'aluminium métallique dans un solvant protique.

2. Procédé selon la revendication 1, **caractérisé en ce que** l'aluminium métallique utilisé est activé par traitement des surfaces par des sels de métaux nobles.

3. Procédé selon la revendication 1 ou 2, **caractérisé en ce que** l'activation de l'aluminium s'effectue à l'aide d'une solution de chlorure de mercure.

4. 5-{4-[2-(5-méthyl-2-(thién-2-yl)-oxazol-4-yl)-éthoxy]-benzo[b]-thiophén-7-ylméthyl}-thiazolidine-2,4-dione
ainsi que ses tautomères, énantiomères, diastéréoisomères et sels physlologiquement acceptables.

5. Médicaments contenant, outre des véhicules et adjuvants usuels, un composé selon la revendication 4.

6. Utilisation des composés selon la revendication 4, pour la fabrication de médicaments destinés au traitement du diabète sucré.
